# EUROPÄISCHE PATENTSCHRIFT

(11) **EP 1 155 138 B1**
(45) Veröffentlichungstag und Bekanntmachung des Hinweises auf die Patenterteilung: **05.01.2005**
(21) Anmeldenummer: 00920443.9
(22) Anmeldetag: 23.02.2000
(51) Int. Cl.: C12P 7/52, C12P 13/02, C12P 41/00, C07C 53/21, C07C 235/06

(54) **VERFAHREN ZUR HERSTELLUNG VON OPTISCH AKTIVEN 3,3,3- TRIFLUORMETHYL- 2- ALKYLPROPIONSÄUREDERIVATEN**
METHOD FOR PREPARING OPTICALLY ACTIVE 3,3,3-TRIFLUOROMETHYL-2-ALKYL PROPIONIC ACID DERIVATIVES
PROCEDE DE PREPARATION DE DERIVES D'ACIDE 3,3,3-TRIFLUOROMETHYLE-2-ALKYLEPROPIONIQUE A ACTIVITE OPTIQUE

(30) Priorität: 23.02.1999 EP 99103420
(43) Veröffentlichungstag der Anmeldung: 21.11.2001
(73) Patentinhaber: Lonza AG, 4052 Basel (CH)
(72) Erfinder: NAUGHTON, Andrew, Mobile, AL 36609 (US); SHAW, Nicholas, CH-3930 Visp (CH); SCHMID, Evelyne, CH-3938 Ausserberg (CH)
(74) Vertreter: Ritthaler, Wolfgang, Dr.rer.nat.Dipl.-Chem.
(86) Internationale Anmeldenummer: PCT/EP2000/001491
(87) Internationale Veröffentlichungsnummer: WO 2000/050623

(56) Entgegenhaltungen:
- WO-A-98/01568
- WO-A-99/04028
- DE-A- 19 725 802
- KURT KONIGSBERGER ET AL.: "The synthesis of (R)- and (S)-alpha-trifluoromethyl-alpha-hydroxycar boxylic acids via enzymatic resolutions" TETRAHEDRON: ASYMMETRY, Bd. 10, Nr. 4, 26. Februar 1999 (1999-02-26), Seiten 679-687, XP002150873

## Beschreibung

Die vorliegende Erfindung betrifft ein neues Verfahren zur Herstellung von optisch aktiven 3,3,3-Trifluor-2-alkylpropionsäurederivaten der allgemeinen Formeln 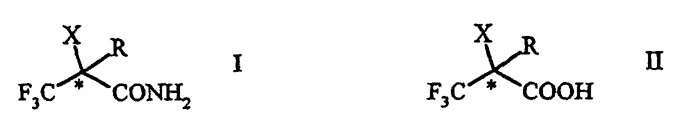 sowie neue Vertreter dieser Verbindungsklasse.

3,3,3-Trifluor-2-alkylpropionsäurederivate, wie bspw. die 3,3,3-Trifluor-2-hydroxy-2-ethylpropionsäure, sind wichtige Zwischenprodukte zur Herstellung von therapeutischen Amiden (EP-A 0 524 781).

Die bekannten 3,3,3-Trifluor-2-alkylpropionsäurederivate, wie bspw. die 3,3,3-Trifluor-2-amino-2-methylpropionsäure, wurden bisher mittels einer Aminoacylase ausgehend von racemischem Trifluoracetyl-R(+)-2-trifluormethylalanin synthetisiert. Dabei wird das Produkt in geringer Ausbeute gebildet.

Aufgabe der vorliegenden Erfindung war ein Verfahren zur Herstellung von optisch aktiven 3,3,3-Trifluor-2-alkylpropionsäurederivaten bereit zu stellen, bei dem die gewünschten Produkte sowohl in hervorragender Ausbeute als auch in relativ kurzen Umsetzungszeiten gebildet werden.

Diese Aufgabe wird mit dem Verfahren nach Anspruch 1 gelöst.

Erfindungsgemäss werden die 3,3,3-Trifluor-2-alkylpropionsäurederivate derart hergestellt, dass man ein racemisches 3,3,3-Trifluor-2-alkylpropionsäureamid der Formel 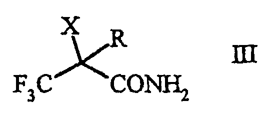 worin R Ethyl oder Methyl und X -OH oder NH₂ bedeutet, ausgenommen, dass wenn R = Methyl, X ≠ OH ist, entweder mittels Mikroorganismen, die befähigt sind, letzteres in Form des Racemats oder eines seiner optisch aktiven Isomere als einzige Stickstoffquelle zu verwerten, oder mittels eines Polypeptids mit Amidohydrolase-Aktivität, welches befähigt ist, letzteres zu hydrolysieren, umsetzt.

Die Edukte, die 3,3,3-Triffuor-2-alkylpropionsäureamide der allgemeinen Formel III, können nach üblichen chemischen Methoden hergestellt werden. Vorzugsweise werden die Edukte, wie bspw. das 3,3,3-Triffuor-2-hydroxy-2-ethylpropionsäureamid, ausgehend von dem entsprechenden 3,3,3-Trifluor-2-alkylpropionsäurenitril der allgemeinen Formel 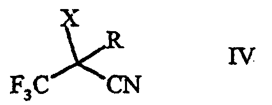 worin R und X die genannte Bedeutung haben, durch Hydrolyse mit einer Mineralsäure hergestellt.

Als Mineralsäure können die fachmännisch üblichen, wie bspw. Phosphorsäure, Salzsäure oder Schwefelsäure, verwendet werden. Vorzugsweise wird als Mineralsäure Schwefelsäure verwendet. Zweckmässig wird die Mineralsäure im Überschuss, vorzugsweise in einer Menge von 2 bis 4 mol pro mol Propionsäurenitril, eingesetzt.

Zweckmässig wird die Hydrolyse bei einer Temperatur von 20 bis 140 °C, vorzugsweise von 90 bis 120 °C, durchgeführt.

Für die erfindungsgemässe Biotransformation können sowohl alle Mikroorganismen, die befähigt sind, das Propionsäureamid der Formel III in Form des Racemats oder eines seiner optisch aktiven Isomere als einzige Stickstoffquelle zu verwerten, als auch alle isolierten Polypeptide mit Amidohydrolase-Aktivität, die befähigt sind, das Propionsäureamid der Formel III zu hydrolisieren, verwendet werden.
Mikroorganismen bzw. Polypeptide mit Amidohydrolase-Aktivität; die diese Eigenschaft besitzen, sowie das isolierte und sequenzierte DNA-Fragment, welches für die Amidohydrolase codiert, sind bereits bekannt und in der WO 98/01568 beschrieben. Als Mikroorganismen können sowohl sog. "Wildstämme", die, wie in der WO 98/01568 beschrieben, aus Bodenproben, Schlamm oder Abwasser isoliert werden können, als auch sog.
"gentechnologisch veränderte Mikroorganismen", die, wie in der WO beschrieben, mit dem isolierten DNA-Fragment transformiert sind, eingesetzt werden. Zweckmässig wird die Biotransformation mittels Mikroorganismen der Gattung Klebsiella oder mittels "gentechnologisch veränderten Mikroorganismen" durchgeführt. Als Mikroorganismen der Gattung Klebsiella werden bevorzugt die der Spezies Klebsiella oxytoca PRS1 (DSM 11009), Klebsiella oxytoca PRS1K17 (DSM 11623), Klebsiella panticula ID-624 (DSM 11354) oder Klebsiella pneumoniae ID-625 (DSM 11355) oder deren funktionell äquivalenten Varianten und Mutanten eingesetzt. Als "gentechnologisch veränderte Mikroorganismen" sind bspw. Mikroorganismen der Spezies Escherichia coli DH5 und Escherichia coli XL-1 Blue MRF, jeweils enthaltend Plasmid pPRS1b, pPRS2a (DSM 11635), pPRS4 oder pPRS7, geeignet. Vorzugsweise wird Escherichia coli XL-1 Blue MRF® enthaltend Plasmid pPRS7 eingesetzt. Die Mikroorganismen mit der Bezeichnung DSM 11009 wurden am 24.06.1996, die mit DSM 11623 am 20.06.1997, die mit DSM 11354 und DSM 11355 am 27.12.1996 und die mit DSM 11635 am 30.06.1997 bei der Deutschen Sammlung von Mikroorganismen und Zellkulturen GmbH, Mascheroderweg 1b, D-38124 Braunschweig, gemäss Budapester Vetrag hinterlegt.

Die Biotransfomation kann nach üblichem Anzüchten der Mikroorganismen mit ruhenden Zellen (nicht wachsende Zellen, die keine C- und Energiequelle mehr benötigen) oder mit wachsenden Zellen durchgeführt werden. Vorzugweise wird die Biotransformation mit ruhenden Zellen durchgeführt.

Für die Biotransformation können fachmännisch übliche Medien wie bspw. niedermolare Phosphatpuffer, HEPES-Puffer oder Mineralsalzmedien eingesetzt werden.

Die Biotransformation wird zweckmässig unter einmaliger oder kontinuierlicher Zugabe an 3,3,3-Trifluor-2-alkylpropionsäureamid der Formel III so durchgeführt, dass die Konzentration 10 Gew. %, vorzugsweise 2,5 Gew. % nicht übersteigt.
Der pH-Wert des Mediums kann in einem Bereich von 4 bis 12, vorzugsweise von 5 bis 11 liegen. Zweckmässig wird die Biotransformation bei einer Temperatur von 10 bis 80 °C, vorzugsweise von 10 bis 50 °C, durchgeführt.

Nach einer üblichen Umsetzungszeit von 1 min bis zu mehreren Tagen können dann die optisch aktiven 3,3,3-Trifluor-2-alkylpropionsäurederivate der allgemeinen Formeln I und II durch übliche Aufarbeitungsmethoden, wie z. B. durch Extraktion, isoliert werden.

Es wurde gefunden, dass wenn man als Substrat das 3,3,3-Trifluor-2-amino-2-methylpropionsäureamid einsetzt, die entsprechende R-Säure innerhalb 5 Minuten gebildet wird und dann das entsprechende S-Amid isoliert werden kann.

Sowohl das racemische 3,3,3-Trifluor-2-hydroxy-2-ethylpropionsäureamid der allgemeinen Formel III als auch die entsprechende (+)-Säure, das entsprechende (-)-Amid und das (S)-3,3,3-Trmuor-2-amino-2-methylpropionsäureamid sind in der Literatur noch nicht beschriebene Verbindungen und demzufolge Bestandteil der Erfindung.

Weiterhin wurde gefunden, dass die optisch aktiven 3,3,3-Trifluor-2-alkylpropionsäureamide der allgemeinen Formel I auf bekannte Weise in die optisch aktiven 3,3,3-Trifluor-2-alkylpropionsäurederivate der allgemeinen Formel II hydrolysiert werden können.
Vorzugsweise wird dabei das (S)-3,3,3-Trifluor-2-amino-2-methylpropionsäureamid in die (S)-3,3,3-Trifluor-2-amino-2-methylpropionsäure hydrolysiert.
Die Hydrolyse erfolgt analog zu der in der WO 98/01568 beschriebenen Hydrolyse. Vorzugsweise erfolgt diese Hydrolyse entweder chemisch in Gegenwart einer Base oder mikrobiologisch mittels Mikroorganismen der Gattung Rhodococcus.

### Beispiel 1

### Herstellung von optisch aktivem 3,3,3-Trifluor-2-hydroxy-2-ethylpropionsäureamid und von optisch aktiver entsprechender Propionsäure

### 1.1 Herstellung von racemischem 3,3,3-Trifluor-2-hydroxy-2-ethylpropionsäureamid

In einen 100-ml Kolben wurde konzentrierte Schwefelsäure (15,3 g) unter Argonatmosphäre hinzugegeben und das Edukt 3,3,3-Trifluor-2-hydroxy-2-ethylpropionsäurenitril (Fluorchem) (8g) tropfenweise hinzugegeben. Die Reaktionsmischung wurde 15 min auf 115 °C erwärmt und dann auf 8 °C abgekühlt und anschliessend wurde destilliertes Wasser (21,8 g) langsam hinzugegeben. Dann wurde Diethylether hinzugefügt und das Ganze 20 min gerührt.
Nach Trennen der Phasen wurde die Ether-Phase mit Wasser (25 ml), mit wässriger gesättigter NaHCO₃ (25 ml) und wieder mit Wasser (25 ml) gewaschen, dann gesammelt, über Na₂SO₄ getrocknet, filtriert und unter Vakuum getrocknet. Das resultierende Öl wurde mit n-Hexan behandelt und über Nacht gerührt. Insgesamt wurden 4,27 g getrocknete Kristalle, entsprechend einer Ausbeute von 48 % erhalten.

### 1.2 Biotransformation

Zellen von E.coli XL-1-Blue MRF/pPRS7 wurden in NYB-Medium (Nutrient Yeast Broth) mit 100 µg/ml Ampicillin bis zu einer optischen Dichte von OD₆₅₀ = 4,74 heranwachsen gelassen. Dann wurden die Zellen in 100 mM Phosphatpuffer (pH 8,0) gewaschen. Die anschliessende Biotransformation wurde mit einer Zellsuspension von einer optischen Dichte von OD₆₅₀ = 10 bei einer Eduktkonzentration (3,3,3-Trifluor-2-hydroxy-2-ethyl-propionsäureamid) von 0,5 % durchgeführt. Das Ganze wurde bei 130 rpm, bei 37 °C, gerührt. Die Biotransfomation wurde mittels chiraler GC gemessen und war nach 125 h beendet.

Um die gebildete R-Säure vom S-Amid, zu trennen, wurde zunächst die Biotransformationslösung von pH 7,8 auf pH 10,0 mit 30 %iger NaOH eingestellt. Dann wurde mit Ethylacetat (600 ml) extrahiert, dann das Ganze über Celite filtriert und die Phasen getrennt. Die dabei erhaltenen wässrigen Phasen wurden mit 30 %iger NaOH auf pH 10,0 eingestellt, mit Ethylacetat abfiltriert und die Phasen getrennt. Das Ganze wurde ca. 3-mal wiederholt. Die vereinigten Extrakte wurden über Na₂SO₄ getrocknet und dann am Rotationsverdampfer eingeengt. Das dabei erhaltene Öl wurde mit Hexan versetzt und auf-18 °C abgekühlt. Nach 12 h wurde die Suspension abfiltriert, mit Hexan gewaschen und getrocknet. Anschliessend wurde das Rohprodukt (ca. 1,8 g) in heissem Toluol umkristallisiert und dann getrocknet. Es wurden 1,72 g (-)-Amid (α_{D} = -13,2, c = 3,75 in Methanol) als beiger Feststoff erhalten, entsprechend einer Ausbeute von 34,4 % und mit einem "enantiomeric excess" (ee) von grösser als 98 %.
Um die gebildete (+)-Säure (α_{D} = +9,75, c = 3,31 in Methanol) zu isolieren, wurde die wässrige Phase von pH 9,4 auf pH 1,0 mit 32 %iger HCl eingestellt und 2-mal mit Ethylacetat extrahiert. Die organischen Phasen wurden vereinigt, mit Na₂SO₄ getrocknet und dann am Rotationsverdampfer eingeengt. Anschliessend wurden zum Rückstand 15 ml Toluol hinzugegeben. Nach dem Einengen wurde 2,18 g bräunlicher Feststoff erhalten. Nach Umkristallisieren in heissem Toluol wurden 1,97 g beiger Feststoff, entsprechend einer Ausbeute von 39,1 % und mit einem ee-Wert von 85,2 %, erhalten.

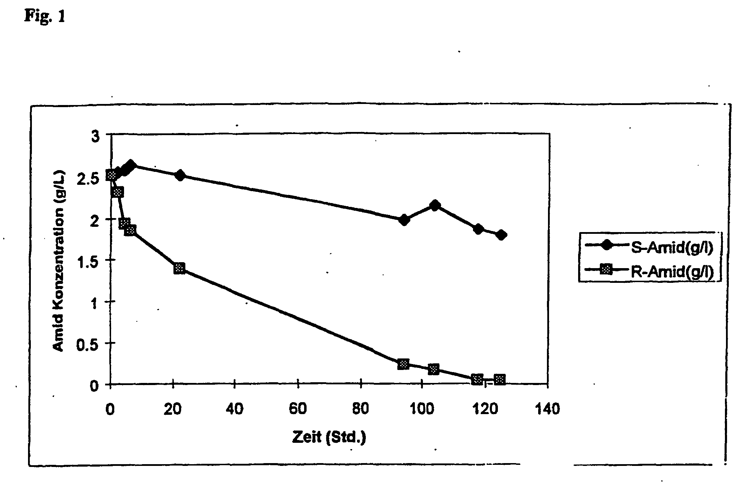

Die Ergebnisse sind in Fig. 1 dargestellt.

### Beispiel 2

### Herstellung von (S)-3,3,3-Trifluor-2-amino-2-methylpropionsäureamid

### 2.1 Herstellung von racemischem 3,3,3-Trifluor-2-amino-2-methylpropionsäureamid

Die Herstellung wurde analog zur EP-A 0 298 029 durchgeführt.
Kaliumcyanid (66,4 g) und destilliertes Wasser (50 ml) wurden vorgelegt und die Suspension auf 0 bis 5 °C abgekühlt. Dann wurde Trifluoraceton (112,1 g) innerhalb 1,5 h hinzugegeben. Die Reaktionsmischung wurde in einen Berghofautoklaven umgefüllt und auf ca. 85 °C erwärmt. Nach ca. 1,5 h wurde Ammoniak-Lösung (124,5 g) hinzugetropft und das Ganze ca. 7 h auf 110 - 115 °C erwärmt.
Die erhaltene braune Lösung wurde am Rotationsverdampfer bei 60 °C eingeengt, um 181,4 g amorphe braune Masse zu erhalten. Das Produkt wurde bei 140 °C/0,1 mbar sublimiert und dann aus Ethylacetat umkristallisiert. Es wurden 0,67 g weisser Feststoff entsprechend einer Ausbeute von 3 % erhalten.

### 2.2 Biotransformation

Die Biotransformation wurde mittels eines zellfreien Enzymextrakts von E.coli XL-1-Blue MRF/pPRS7 durchgeführt. Dazu wurden die Zellen in NYB mit 100 µg/ml Ampicillin bis zu einer optischen Dichte von OD₆₅₀ = 3,59 angezüchtet. Dann wurden sie in 100 mM Phosphatpuffer gewaschen und dann im selben Puffer zu einer OD₆₅₀ von 19 resuspendiert. Die Zellen wurden durch 3-maliges Behandeln mit der French-Presse aufgebrochen, der Zellextrakt 5 min auf 75 °C erhitzt und die Zelltrümmer durch Zentrifugation (20'000 x g) entfernt. Der klare Überstand hatte eine Proteinkonzentration von 9,75 mg/ml und wurde für die Biotransfomation eingesetzt. Hierzu wurde 0,5 % racemisches 3,3,3-Trifluor-2-amino-2-methylpropionsäureamid bei 40 °C mit 0,9 mg/ml Enzymextrakt versetzt. Die Reaktion wurde mittels chiraler GC verfolgt. Nach 5 min war die Umsetzung vollständig.
Die Reaktion wurde durch Zugabe des gleichen Volumens an Ethylacetat gestoppt und dann 4-mal mit Ethylacetat extrahiert.
Um das (S)-Amid zu isolieren, wurde die wässrige Phase mit 200 ml Ethylacetat extrahiert (4-mal) und die vereinigten Extrakte mit NaSO₄ getrocknet und anschliessend am Rotationsverdampfer bei 40 °C eingeengt. Es wurden 640 mg gelbbrauner Feststoff erhalten. Dieser Feststoff wurde aus 0,5 g Ethylacetat und Hexan (4 ml) umkristallisiert. Es wurden 0,5 g (S)-Amid entsprechend einer Ausbeute von 38,5 % bez. eingesetzten Racemats erhalten. Der ee-Wert betrug 100.

## Patentansprüche

1. Verfahren zur Herstellung von optisch aktiven 3,3,3-Trifluor-2-alkylpropionsäurederivaten der allgemeinen Formeln 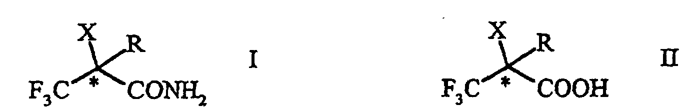 worin R Ethyl oder Methyl und X -OH oder NH₂ bedeutet, ausgenommen, dass wenn R = Methyl, X ≠ -OH ist, umfassend die Umsetzung eines racemischen 3,3,3-Trifluor-2-alkylpropionsäureamids der allgemeinen Formel 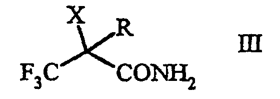 worin R und X die genannte Bedeutung haben, entweder mittels Mikroorganismen, die befähigt sind, letzteres in Form des Racemats oder eines seiner optisch aktiven Isomere als einzige Stickstoffquelle zu verwerten, oder mittels eines Polypeptids mit Amidohydrolase-Aktivität, welches befähigt ist, letzteres zu hydrolysieren.

2. Verfahren nach Anspruch 1, **dadurch gekennzeichnet, dass** man das 3,3,3-Trifluor-2-alkylpropionsäureamid der allgemeinen Formel 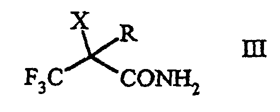 derart herstellt, dass ein 3,3,3-Trifluor-2-alkylpropionsäurenitril der allgemeinen Formel 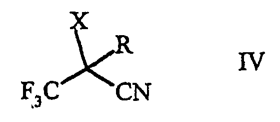 mit einer Mineralsäure hydrolysiert wird.

3. Verfahren nach Anspruch 2, **dadurch gekennzeichnet, dass** als Mineralsäure Schwefelsäure eingesetzt wird.

4. Verfahren nach Anspruch 2 oder 3, **dadurch gekennzeichnet, dass** die Hydrolyse bei einer Temperatur von 20 bis 140 °C durchgeführt wird.

5. Verfahren nach mindestens einem der Ansprüche 1 bis 4, **dadurch gekennzeichnet, dass** die Biotransformation mittels Mikroorganismen der Gattung Klebsiella durchgeführt wird.

6. Verfahren zur Herstellung von optisch aktiven 3,3,3-Trifluor-2-alkylpropionsäurederivaten der allgemeinen Formel 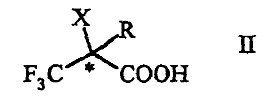 worin R Ethyl oder Methyl und X -OH oder NH₂ bedeutet, ausgenommen, dass wenn R = Methyl, X ≠ OH ist, **dadurch gekennzeichnet, dass** in der ersten Stufe ein racemisches 3,3,3-Trifluor-2-alkylpropionsäureamid der allgemeinen Formel 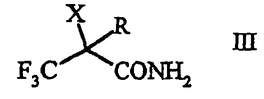 worin R und X die genannte Bedeutung haben, entweder mittels Mikroorganismen, die befähigt sind, letzteres in Form des Racemats oder eines seiner optisch aktiven Isomere als einzige Stickstoffquelle zu verwerten, oder mittels eines Polypeptids mit Amidohydrolase-Aktivität, welches befähigt ist, letzteres zu hydrolysieren, in ein optisch aktives 3,3,3-Trifluor-2-alkylpropionsäureamid der allgemeinen Formel 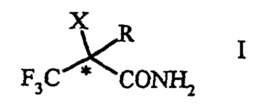 worin R und X die genannte Bedeutung haben, überführt wird und letzteres dann in der zweiten Stufe auf bekannte Weise in das optisch aktive 3,3,3-Trifluor-2-alkylpropionsäurederivat (Formel II) hydrolysiert wird.

7. 3,3,3 -Trifluor-2-hydroxy-2-ethylpropionsäureamid der Formel

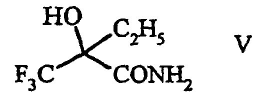

8. (+)-3,3,3-Trifluor-2-hydroxy-2-ethylpropionsäure

9. (-)-3,3,3-Trifluor-2-hydroxy-2-ethylpropionsäureamid

10. (S-)-3,3,3-Trifluor-2-amino-2-methylpropionsäureamid

## Claims

1. A process for preparing optically active 3,3,3-tnfluoro-2-alkylpropionic acid derivatives of the general formulae 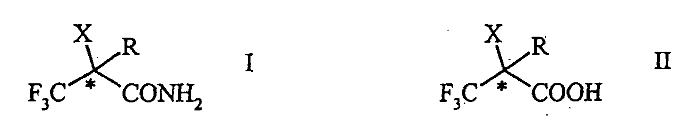 in which R denotes ethyl or methyl and X denotes -OH or NH₂, with the exception that X≠OH when R=methyl, comprising the transformation of a racemic 3,3,3-trifluoro-2-alkylpropionamide of the general formula 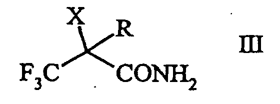 in which R and X have said meaning, either using microorganisms which are capable of utilizing the latter, as their sole source of nitrogen, in the form of the racemate or of one of its optically active isomers, or using a polypeptide which possesses amidohydrolase activity and which is capable of hydrolyzing the latter.

2. The process as claimed in claim 1, wherein the racemic 3,3,3-trifluoro-2-alkylpropionamide of the general formula 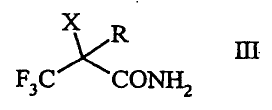 is prepared by hydrolyzing a 3,3,3-tnfluoro-2-alkylpropionomtrile of the general formula 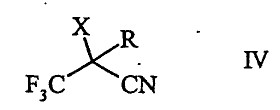 with a mineral acid.

3. The process as claimed in claim 2, **characterized in that** the mineral acid employed is sulfuric acid.

4. The process as claimed in claims 2, **characterized in that** the hydrolysis is carried out at a temperature of from 20 to 140°C.

5. The process as claimed in claims 1, **characterized in that** the biotransformation is carried out using microorganisms of the genus Klebsiella.

6. A process for preparing optically active 3,3,3-trifluoro-2-alkylpropionic acid derivatives of the general formula 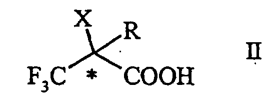 in which R denotes ethyl or methyl and X denotes -OH or NH₂, with the exception that X≠OH when R=methyl, **characterized in that**, in the first step, a racemic 3,3,3-trifluoro-2-alkylpropionamide of the general formula 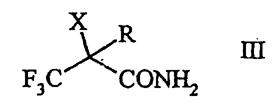 in which R and X have said meaning, is transformed, either using microorganisms which are capable of utilizing the latter, as their sole source of nitrogen, in the form of the racemate or of one of its optically active isomers, or using a polypeptide which possesses amidohydrolase activity and which is capable of hydrolyzing the latter, into an optically active 3,3,3-trifluoro-2-alkylpropionamide of the general formula 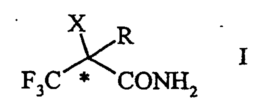 in which R and X have said meaning, and the latter is then hydrolyzed, in the second step and in a known manner, into the optically active 3,3,3-trifluoro-2-alkylpropionic acid derivative (formula II).

7. 3,3,3-Trifluoro-2-hydroxy-2-ethylpropionamide of formula

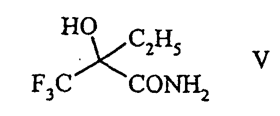

8. (+)-3,3,3-Trifluoro-2-hydroxy-2-ethylpropionic acid.

9. (-)-3,3,3-Thifluoro-2-hydroxy-2-ethylpropionamide.

10. (S)-3,3,3-Trifluoro-2-amino-2-methylpropionamide.

## Revendications

1. Procédé pour la préparation de dérivés optiquement actifs d'acide 3,3,3-trifluoro-2-alkylpropionique de formules générales 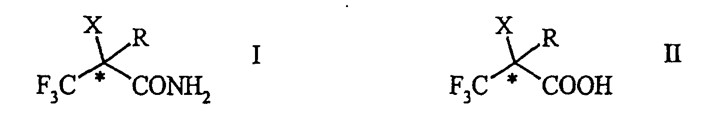 dans laquelle R représente un groupe éthyle ou méthyle et X représente -OH ou NH₂, étant exclu que lorsque R = méthyle, X soit ≠ -OH, comprenant la conversion d'un 3,3,3-trifluoro-2-alkylpropionamide racémique de formule générale 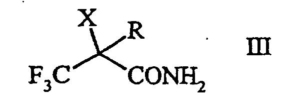 dans laquelle R et X ont les significations données, soit au moyen de micro-organismes qui sont capables d'utiliser comme seule source d'azote ce dernier sous forme du racémique ou d'un de ses isomères optiquement actifs, soit au moyen d'un polypeptide à activité d'amidohydrolase, qui est capable d'hydrolyser ce dernier.

2. Procédé selon la revendication 1, **caractérisé en ce qu'**on prépare le 3,3,3-trifluoro-2-alkylpropionamide de formule générale 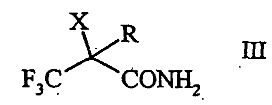 en hydrolysant à l'aide d'un acide minéral un 3,3,3-trifluoro-2-alkylpropionitrile de formule générale

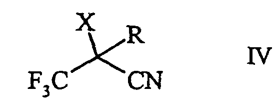

3. Procédé selon la revendication 2, **caractérisé en ce qu'**on utilise comme acide minéral l'acide sulfurique.

4. Procédé selon la revendication 2 ou 3, **caractérisé en ce qu'**on effectue l'hydrolyse à une température de 20 à 140°C.

5. Procédé selon au moins l'une des revendications 1 à 4, **caractérisé en ce que** la biotransformation est effectuée au moyen de micro-organismes appartenant au genre *Klebsiella*.

6. Procédé pour la préparation de dérivés optiquement actifs d'acide 3,3,3-trifluoro-2-alkylpropionique de formule générale 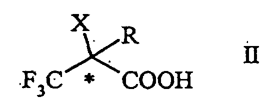 dans laquelle R représente un groupe éthyle ou méthyle et X représente -OH ou NH₂, étant exclu que lorsque R = méthyle, X soit ≠ -OH, **caractérisé en ce que** dans la première étape un 3,3,3-trifluoro-2-alkylpropionamide racémique de formule générale 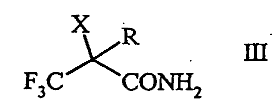 dans laquelle R et X ont les significations données, est converti, soit au moyen de micro-organismes qui sont capables d'utiliser comme seule source d'azote ce dernier sous forme du racémique ou d'un de ses isomères optiquement actifs, soit au moyen d'un polypeptide à activité d'amidohydrolase, qui est capable d'hydrolyser ce dernier, en un 3,3,3-trifluoro-2-alkylpropionamide optiquement actif de formule générale 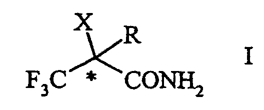 dans laquelle R et X ont les significations données, et ce dernier, dans la seconde étape, est ensuite hydrolysé de façon connue en la dérivé optiquement actif d'acide 3,3,3-trifluoro-2-alkylpropionique (formule II).

7. 3,3,3-Trifluoro-2-hydroxy-2-éthylpropionamide de formule

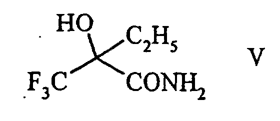

8. Acide (+)-3,3,3-trifluoro-2-hydroxy-2-éthylpropionique.

9. (-)-3,3,3-Trifluoro-2-hydroxy-2-éthylpropionamide.

10. (S)-3,3,3-Trifluoro-2-amino-2-méthylpropionamide.
